# EUROPÄISCHE PATENTSCHRIFT

(11) **EP 2 844 170 B1**
(45) Veröffentlichungstag und Bekanntmachung des Hinweises auf die Patenterteilung: **01.02.2017**
(21) Anmeldenummer: 13703822.0
(22) Anmeldetag: 13.02.2013
(51) Int. Cl.: A61B 17/86

(54) **KNOCHENSCHRAUBE**
BONE SCREW
VIS OSSEUSE

(30) Priorität: 04.05.2012 AT 5302012
(43) Veröffentlichungstag der Anmeldung: 11.03.2015
(73) Patentinhaber: Pastl, Klaus, 4040 Lichtenberg (AT)
(72) Erfinder: Pastl, Klaus, 4040 Lichtenberg (AT)
(74) Vertreter: KLIMENT & HENHAPEL
(86) Internationale Anmeldenummer: PCT/EP2013/052869
(87) Internationale Veröffentlichungsnummer: WO 2013/164107

(56) Entgegenhaltungen:
- EP-A1- 2 384 712
- US-A1- 2009 312 842

## Beschreibung

Die Erfindung betrifft einen Bolzen mit Außengewinde und einem zylindrischen Bolzenschaft aus allogenem, kortikalen Knochenmaterial für die chirurgisch operative Osteosynthese, wobei das Knochenmaterial mit Havers-Kanälen durchzogen ist, gemäß dem Oberbegriff von Anspruch 1, sowie ein Verfahren zur Herstellung einer solchen Knochenschraube gemäß dem Oberbegriff von Anspruch 2. Sofern sich das Außengewinde eines solchen Bolzens über die gesamte Längserstreckung des Bolzens erstreckt, kann auch von einem Gewindestift gesprochen werden. Ist der Bolzen ferner mit einem gewindelosen Kopf versehen, kann auch von einer Knochenschraube gesprochen werden. Im Folgenden wird vorzugsweise von Knochenschrauben gesprochen, wobei von dieser Bezeichnung auch Ausführungen in Form eines Gewindestifts umfasst sein sollen.

Knochenschrauben für die chirurgisch operative Osteosynthese werden in herkömmlicher Weise aus Metall bzw. Metalllegierungen gefertigt. Ferner sind Knochenschrauben aus resorbierbarem Material, etwa Polyglykolid und Polylaktid, bekannt, sowie Schrauben aus xenogenem Knochen. Knochenschrauben dieser Art weisen in der chirurgischen Praxis allerdings mehrere Nachteile auf. So müssen etwa Schrauben aus Metall bzw. Metalllegierungen einerseits durch eine Zweitoperation wieder entfernt werden, und unterliegen andererseits Veränderungen durch Korrosion. Damit erhöhen sich die Kosten im Gesundheitssystem. Außerdem besteht ein zusätzliches gesundheitliches Risiko für jeden Patienten durch eine neuerliche Operation, das bei Schrauben aus allogenem Knochen nicht besteht. Sämtliche resorbierbaren Materialien im menschlichen oder tierischen Körper wiederum bilden zwar je nach Material eine mehr oder weniger feste Brücke zwischen den zu osteosynthetisierenden Knochen, werden aber aufgelöst, was die Festigkeit der Osteosynthese der betroffenen Knochen negativ beeinflusst. Des Weiteren führen manche resorbierbare Synthesematerialien während ihres Abbaus zu großen Osteolysen in dem umgebenden Knochen, also zu einem Wegweichen des Empfängerknochens von der Schraube. Xenogene (speziesfremde) Materialien wiederum führen zu Abstoßungsreaktionen und sind für die Osteosynthese auch deshalb ungeeignet, weil sie in den umgebenden Empfängerknochen nicht eingebaut, sondern abgestoßen und abgebaut werden, auch wenn das Eiweiß im Knochen vorher durch Hitze denaturiert wurde. Weiters trägt auch der unterschiedliche Elastizitätsmodul von boviner Corticalis und humaner Corticalis (Mensch ca. 16.000 N/mm², Rind ca. 22.000 bis 24.000 N/mm²) dazu bei, dass humanes Material wesentlich besser einheilen kann. Formfestigkeit und Elastizitätsmodul des corticalen Knochens sind dabei speziesabhängig.

Schrauben aus allogenem Knochen (Femur und Tibia-Corticalis) verfügen hingegen über mehrere Vorteile. Sie werden ohne Abstoßungsreaktion vaskularisiert und umgebaut, und sind vor allem für Osteosynthesen dort geeignet, wo kleine Knochenfragmente zusammengefügt werden müssen, da durch die Schraube bereits bei der Operation eine tragende Knochenbrücke entsteht, die sich vom Zeitpunkt der Operation an verbessert, indem sie sich umbaut und voll in den lebenden Knochen integriert und eingebaut wird. Im Gegensatz dazu stellen Metallschrauben eher ein Hindernis für die Knochenneubildung dar, insbesondere verringern sie durch deren bloße Präsenz die zur Verfügung stehende Oberfläche, die für die Knochenheilung vorhanden wäre. Abbaubare Materialien wiederum haben ihre maximale Festigkeit zum Zeitpunkt der Operation. Für sie gelten dieselben Nachteile wie für die Metallschrauben, des Weiteren nimmt die Festigkeit rapide ab, sobald der Abbauprozess eintritt, wodurch die zu osteosynthetisierende Knochenstelle zumindest vorübergehend wieder eine Schwächung erfährt.

Des Weiteren kann bei Knochenschrauben aus allogenem Knochen eine Zweitoperation zur Entfernung des Osteosynthesematerials entfallen, da der Knochen vollständig in eigenen Knochen umgewandelt (nicht resorbiert!) wird. Für den Patienten verringert sich somit das Operationsrisiko, für das Gesundheitssystem verringern sich gezwungenermaßen die Kosten. Schrauben aus allogenem Knochen stören auch nicht bei der Anwendung bildgebender Verfahren, im Gegensatz zu Metallschrauben, die störende Artefakte im MRI und CT hinterlassen. Auch Nachuntersuchungen sind problemlos möglich, und lassen eine bessere Beurteilung des Heilungserfolges zu.

Schrauben aus allogenem, kortikalen Knochenmaterial für die chirurgisch operative Osteosynthese wurde insbesondere in der EP 2 384 712 A des Anmelders beschrieben. In der US 2009/0312842 A1 wurden Implantate für osteochondrale Transplantationen beschrieben.

Allerdings ist es für eine Anwendbarkeit von Schrauben aus allogenem Knochen in der chirurgischen Praxis notwendig, sie hinsichtlich Eindrehwiderstand und Festigkeit zu optimieren. Knochenschrauben aus allogener, humaner Corticalis sind an sich sehr spröde und können bei ungeeignetem Ausgangsmaterial bei Torsions- und Zugbelastungen leicht brechen. Belastungen der Knochenschraube treten aber nicht nur beim Eindrehen der Knochenschraube im Zuge der Operation auf, sondern auch in der postoperativen Heilungsphase. Da die gattungsgemäßen Knochenschrauben außerdem aus allogener, humaner Corticalis gewonnen werden, unterliegt das Ausgangsmaterial in seinen Eigenschaften Schwankungen, die sich auf die Qualität der aus diesem Ausgangsmaterial gefertigten Knochenschraube auswirken. Diese Schwankungen treten dabei nicht nur innerhalb ein- und desselben Knochens auf, sondern auch im Knochenmaterial unterschiedlicher Spender. Tatsächlich besteht hierin auch ein Grund dafür, dass Schrauben aus autologem oder allogenem Knochen bislang in der chirurgischen Praxis noch wenig Verbreitung gefunden haben.

Es ist daher das Ziel der Erfindung, Schrauben aus allogenem, kortikalen Knochen so auszuführen, dass sie über eine größtmögliche Festigkeit verfügen.

Dieses Ziel wird durch die Merkmale von Anspruch 1 erreicht. Anspruch 1 bezieht sich auf einen Bolzen mit Außengewinde und einem zylindrischen Bolzenschaft aus allogenem, kortikalen Knochenmaterial für die chirurgisch operative Osteosynthese, wobei das Knochenmaterial mit Havers-Kanälen durchzogen ist. Ein Havers-Kanal ist der zentrale Knochenkanal in der Mitte eines Osteons, das das Grundbauelement der Kortikalis ist. Ein Osteon besteht dabei aus konzentrisch angeordneten Lamellen, die auch als Speziallamellen bezeichnet werden, einem zentral gelegenen Havers-Kanal und den, zwischen den Lamellen liegenden Osteozyten. Im Havers-Kanal verlaufen Blutgefäße, Bindegewebszellen und - fasern sowie einzelne Nervenfasern.

Osteone und Havers-Kanäle kommen nur in der Kortikalis langer Röhrenknochen vor, wobei die Havers-Kanäle im Wesentlichen in Längsrichtung des Knochens verlaufen. Beim Zuschnitt von Knochenschrauben wird in herkömmlicher Weise der Symmetrie des Knochenaufbaus gefolgt und die Knochenschraube in Längsrichtung des Spenderknochens geschnitten, sodass die Längsachse des Bolzenschaftes im Wesentlichen in Längsrichtung des Spenderknochens verläuft. Somit verlaufen auch die Havers-Kanäle im Bolzenschaft im Wesentlichen parallel zur Längsachse des Bolzenschaftes. Erfindungsgemäß ist nun vorgesehen, dass in einem Querschnitt normal zur Längsachse des Bolzenschaftes die Gesamtfläche der Havers-Kanäle höchstens 10% der Gesamtfläche des Querschnittes einnimmt.

Es wurde nämlich vom Anmelder festgestellt, dass die mechanischen Eigenschaften von Knochenschrauben aus allogenem, kortikalen Knochenmaterial zu einem beträchtlichen Teil von der verfügbaren Knochenmatrix abhängen. Eine flächenmäßige Zunahme der Havers-Kanäle im Querschnitt eines Knochens reduziert dabei die verfügbare Knochenmatrix, wobei der flächenmäßige Anteil von Havers-Kanälen im Knochenquerschnitt nicht nur entlang der Längserstreckung des Knochens variiert, sondern auch zwischen dem Knochenmaterial unterschiedlicher Spender. Wie im Folgenden noch näher ausgeführt wird, besteht ein starker Zusammenhang zwischen dem flächenmäßigen Anteil der Havers-Kanäle im Spenderknochen und der Festigkeit der aus dem Spenderknochen gefertigten Knochenschraube.

Das erfindungsgemäße Kriterium stellt ein einfaches und leicht messbares Kriterium für die Auswahl des Ausgangsmaterials für die Herstellung von Knochenschrauben dar, das außerdem eine gute Qualität der Knochenschraube in verlässlicher Weise sicherstellt. In entsprechender Weise wird erfindungsgemäß auch ein Verfahren zur Herstellung eines Bolzens mit Außengewinde und einem zylindrischen Bolzenschaft aus allogenem, kortikalen und mit Havers-Kanälen durchzogenen Knochenmaterial eines Spenderknochens für die chirurgisch operative Osteosynthese vorgeschlagen, wobei in einem ersten Schritt der Spenderknochen durch Schnitte quer zu seiner Längserstreckung in mehrere Teilstücke unterteilt wird, aus denen in einem zweiten Verfahrensschritt der Bolzenschaft der Knochenschrauben in einer, zu den Havers-Kanälen im Wesentlichen parallelen Richtung geschnitten wird, und in einem dritten Schritt der Bolzenschaft mit einem Außengewinde versehen wird. Erfindungsgemäß wird hierbei vorgeschlagen, dass ausschließlich jene Teilstücke dem zweiten Verfahrensschritt unterzogen werden, deren Grund- und Deckfläche eine Gesamtfläche der Havers-Kanäle von höchstens 10% der Gesamtfläche der jeweiligen Grund- oder Deckfläche aufweist. In der mikroskopischen Analyse der Grund- und Deckfläche eines solchen Teilstückes kann dieses Kriterium selbstredend anhand einer Einheitsfläche im Okular des Mikroskops untersucht werden.

Es ist somit lediglich der Spenderknochen entlang seiner Längserstreckung in mehrere Teilstücke mit einer Höhe von mehreren Zentimetern jeweils mit einem Schnitt quer zur Längsachse des Knochens zu zerteilen, und die Grund- und Deckfläche der einzelnen Teilstücke mikroskopisch zu untersuchen. Die Havers-Kanäle sind in der mikroskopischen Betrachtung als dunkle Flecken ersichtlich, deren Gesamtfläche entweder mithilfe geeigneter Auswertesoftware automatisch ermittelt werden kann, oder mit bloßem Auge mithilfe eines Rasters im Okular des Mikroskops.

Die Erfindung wird im Folgenden anhand eines Ausführungsbeispiels mithilfe der beiliegenden Figuren näher erläutert. Es zeigen hierbei die
Fig. 1 eine schematische Darstellung einer Knochenschraube,
Fig. 2 eine schematische Darstellung der Bruchstelle eines Knochens mit einem eingesetzten Bolzen gemäß der Erfindung im Rahmen einer operativen Osteosynthese,
Fig. 3 ein Ausschnitt eines Querschnittes quer zur Längsachse eines Spenderknochens zur Illustration des Aufbaus eines Osteons und seines Havers-Kanal,
Fig. 4a eine schematische Darstellung eines erfindungsgemäßen Bolzens im Empfängerknochen mit eingezeichnetem Verlauf der Havers-Kanäle gemäß einer ersten Ausführungsform,
Fig. 4b eine schematische Darstellung eines erfindungsgemäßen Bolzens im Empfängerknochen mit eingezeichnetem Verlauf der Havers-Kanäle gemäß einer weiteren Ausführungsform, und die
Fig. 5 eine schematische Darstellung eines Spenderknochens zur Erläuterung des erfindungsgemäßen Verfahrens.

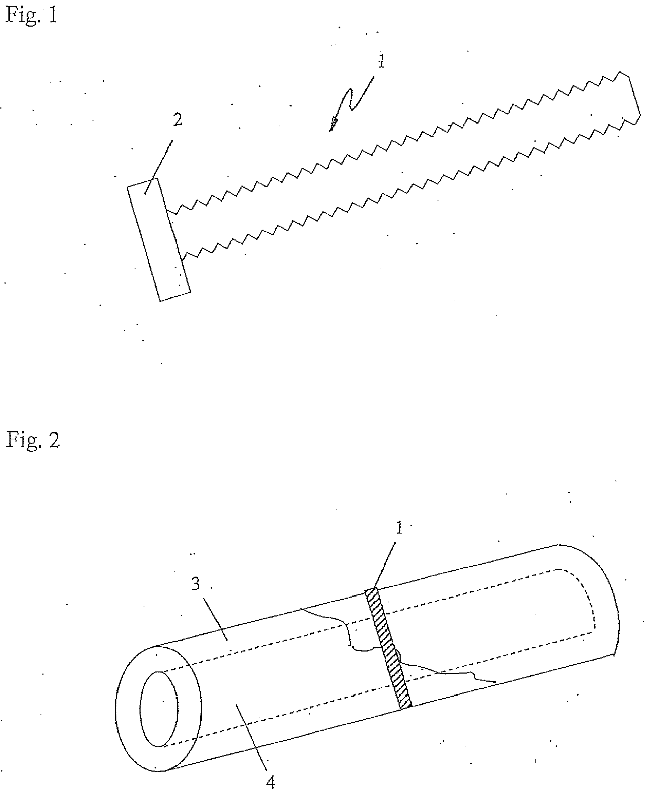

Die vorliegende Erfindung betrifft die Optimierung von Knochenschrauben 1 aus allogenem Knochen, wie sie schematisch in der Fig. 1 dargestellt ist, hinsichtlich ihrer Eigenschaften im Rahmen der operativen Osteosynthese, insbesondere in Bezug auf ihre Biege- und Scherfestigkeit, sowie ihr Bruchdrehmoment.

Vorzugsweise sind hierfür Knochenschrauben mit symmetrischem Spitzgewinde vorgesehen, um keine Kompression auf die zu verbindenden Knochenteile auszuüben. Da außerdem die Festigkeit der Knochenverbindung im Wesentlichen von der Anzahl der Gewindegänge in der Kortikalis 3 des Empfängerknochens abhängt (siehe Fig. 2), und der Beitrag der Verschraubung entlang des spongiösen Knochens 4 nur gering ist, werden die Knochenschrauben vorzugsweise so ausgeführt, dass sie eine möglichst hohe Anzahl an Gewindegängen pro Millimeter entlang des gesamten Bolzenschaftes aufweisen um sicherzustellen, dass die Kortikalis 3 in Verbindung mit einer entsprechend hohen Zahl an Gewindegängen steht. Bei einem Verhältnis von Gewindetiefe zu Gewindedurchmesser zwischen 0.10 und 0.15 ist hierzu etwa ein Produkt von dieser Verhältniszahl und der Anzahl der Gewindegänge pro Millimeter von 0.10 bis 0.30 vorgesehen. Das symmetrische Spitzgewinde wird außerdem über die gesamte Länge des Bolzenschaftes mit konstantem Gewindedurchmesser geführt, sodass sich eine Knochenschraube 1 wie in der Fig. 1 dargestellt ergibt. Die Knochenschraube 1 selbst kann einen Vierkant, Sechskant oder Sternkopf als Schraubenkopf 2 aufweisen (siehe Fig. 1), der aber lediglich dem Einbringen von Drehmoment im Zuge des Einschraubens dient, und nach Platzierung der Knochenschraube 1 abgeschnitten wird, sodass eine Konfiguration gemäß Fig. 2 erreicht wird.

Im Zuge der operativen Osteosynthese wird zunächst die Fixierung und Kompression der zu verbindenden Knochenteile durch entsprechende chirurgische Instrumente sichergestellt. In weiterer Folge wird eine Kernbohrung gesetzt, in das ein Gewinde geschnitten wird. Die erfindungsgemäße Knochenschraube 1 kann nun etwa mit einem Steckschraubenschlüssel mit Drehmoment eingebracht werden, wobei die Knochenschraube 1 die Knochenteile entlang des spongiösen Knochens 4 und der Kortikalis 3 quert, und in einem stabilen Zustand mit vorgegebener Distanz hält, ohne dabei eine Kompression auszuüben, da ein symmetrisch ausgeführtes Spitzgewinde kaum Zugbelastungen zulässt (siehe Fig. 2). Die dargestellte Knochenschraube 1 stellt daher keine Zugschraube dar. Aufgrund der hohen Gewindezahl und der damit verbundenen Reibungskräfte ist aber dennoch hohe Rotationsstabilität der Knochenschraube 1 gegeben.

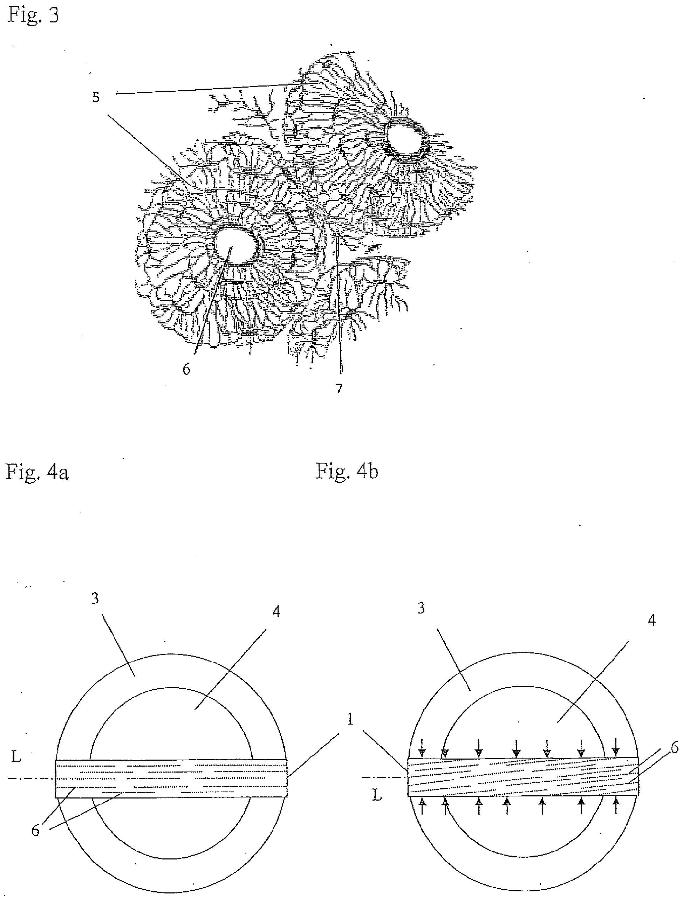

Zur Erläuterung der erfindungsgemäßen Merkmale wird nun zunächst auf die Fig. 3 Bezug genommen. Die Fig. 3 zeigt einen Ausschnitt eines Querschnittes quer zur Längsachse eines Spenderknochens zur Illustration des Aufbaus eines Osteons 5 und seines Havers-Kanal 6. Ein Osteon 5 besteht dabei aus konzentrisch angeordneten Lamellen, die auch als Speziallamellen bezeichnet werden, dem zentral gelegenen Havers-Kanal 6 und den, zwischen den Lamellen liegenden Osteozyten. Im Havers-Kanal 6 verlaufen Blutgefäße, Bindegewebszellen und -fasern sowie einzelne Nervenfasern. Die einzelnen Osteone 5 sind wiederum durch sogenannte Schaltlamellen 7 miteinander verbunden, wobei die Havers-Kanäle 6 der einzelnen Osteone 5 durch, ebenfalls Blutgefäße enthaltende Querkanäle untereinander verbunden sind, die auch als Volkmann-Kanäle bezeichnet werden (in der Fig. 3 nicht ersichtlich).

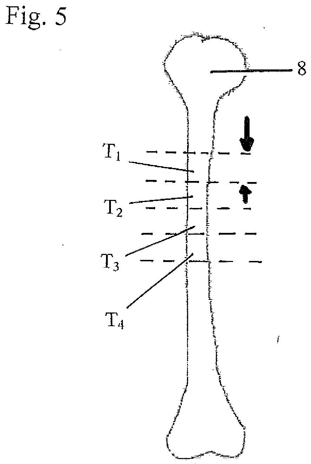

Osteone 5 und Havers-Kanäle 6 kommen nur in der Kortikalis 3 langer Röhrenknochen vor, wobei die Havers-Kanäle 6 im Wesentlichen in Längsrichtung des Spenderknochens 8 verlaufen. Beim Zuschnitt von Knochenschrauben 1 wird der Spenderknochen 8 in herkömmlicher Weise durch Schnitte quer zu seiner Längserstreckung in mehrere Teilstücke T_{N} unterteilt (siehe auch Fig. 5), aus denen in einem zweiten Verfahrensschritt der Bolzenschaft der Knochenschrauben 1 in einer, zu den Havers-Kanälen 6 im Wesentlichen parallelen Richtung geschnitten wird. Somit verlaufen auch die Havers-Kanäle 6 im Bolzenschaft der Knochenschraube 1 im Wesentlichen parallel zur Längsachse L des Bolzenschaftes, wie anhand der Fig. 4a gezeigt ist. Im Rahmen der vorliegenden Erfindung umfasst die Formulierung "im Wesentlichen parallel zur Längsachse L des Bolzenschaftes" auch jenen Fall, wie er in der Fig. 4b dargestellt ist, bei dem die Havers-Kanäle 6 in einem maximalen Winkel von 15° schräg zur Längsachse L des Bolzenschaftes der Knochenschraube 1 verlaufen. Auf diese Weise wird erreicht, dass sich vermehrt Öffnungen von Havers-Kanälen 6 entlang der Mantelfläche des Bolzenschaftes befinden, sodass sie für einen Stoffwechselaustausch mit dem Empfängerknochen zugänglich sind, wie anhand der kleinen Pfeile normal auf die Mantelfläche des Bolzenschaftes in der Fig. 4b angedeutet wird. Die Integration der Knochenschraube 1 im Empfängerknochen wird dadurch wesentlich beschleunigt, da die Integration der Knochenschraube 1 in den Empfängerknochen und ihre Umwandlung in eigenen Knochen zum beträchtlichen Teil über die Havers-Kanäle 6 verläuft. In weiterer Folge wird in einem dritten Schritt der Bolzenschaft mit einem Außengewinde versehen.

Erfindungsgemäß ist nun vorgesehen, dass ausschließlich jene Teilstücke dem zweiten Verfahrensschritt zur Herstellung einer erfindungsgemäßen Knochenschraube 1 unterzogen werden, deren Grund- und Deckfläche eine Gesamtfläche der Havers-Kanäle 6 von höchstens 10% der Gesamtfläche der jeweiligen Grund- oder Deckfläche aufweist. In der mikroskopischen Analyse der Grund- und Deckfläche eines solchen Teilstückes kann dieses Kriterium selbstredend anhand einer Einheitsfläche im Okular des Mikroskops untersucht werden. Eine mithilfe des erfindungsgemäßen Verfahrens hergestellte Knochenschraube 1 weist somit das erfindungsgemäße Merkmal auf, dass in einem Querschnitt normal zur Längsachse L des Bolzenschaftes die Gesamtfläche der Havers-Kanäle 6 höchstens 10% der Gesamtfläche des Querschnittes einnimmt. Selbstredend könnte auch bei einer Knochenschraube 1 dieses Kriterium anhand einer Einheitsfläche im Okular des Mikroskops untersucht werden.

Es ist somit lediglich der Spenderknochen 8 entlang seiner Längserstreckung in mehrere Teilstücke T_{N} mit einer Höhe von mehreren Zentimetern, etwa 5-10cm, jeweils mit einem Schnitt quer zur Längserstreckung des Spenderknochens 8 zu zerteilen, wie es anhand der strichlierten Linien in der Fig. 5 angedeutet ist, und die Grund- und Deckfläche der einzelnen Teilstücke T_{N} entlang der Pfeile der Fig. 5 mikroskopisch zu untersuchen. In der Fig. 5 sind etwa vier Teilstücke T₁, T₂, T₃ und T₄ dargestellt. Die Havers-Kanäle 6 sind in der mikroskopischen Betrachtung als dunkle Flecken ersichtlich, deren Gesamtfläche wie oben erwähnt wurde entweder mithilfe geeigneter Auswertesoftware automatisch ermittelt werden kann, oder mit bloßem Auge mithilfe eines Rasters im Okular des Mikroskops.

Teilstücke T_{N} des Spenderknochens 8, deren Grund- und Deckfläche eine Gesamtfläche der Havers-Kanäle 6 von 10% der Gesamtfläche der jeweiligen Grund- oder Deckfläche übersteigt, werden nicht zur Herstellung von Knochenschrauben 1 herangezogen. Nur jene Teilstücke T_{N} des Spenderknochens 8, deren Grund- und Deckfläche eine Gesamtfläche der Havers-Kanäle 6 von höchstens 10% der Gesamtfläche der jeweiligen Grund- oder Deckfläche aufweist, wird nach dem erfindungsgemäßen Verfahren zur Herstellung von Knochenschrauben 1 verwendet. Auf diese Weise können die durchschnittlichen Biege- und Scherfestigkeiten, sowie Bruchdrehmomente der erfindungsgemäßen Knochenschrauben 1 erhöht werden, und für die einzelne Knochenschraube 1 Mindestwerte für ihre Biege- und Scherfestigkeit, sowie ihr Bruchdrehmoment sichergestellt werden. Dieser Umstand ist für den praktischen Einsatz überaus wichtig, da bislang aufgrund des qualitativ unterschiedlichen Materials von Spenderknochen 8 unterschiedlicher Spender und des qualitativ unterschiedlichen Materials entlang desselben Spenderknochens 8 die Qualität der Knochenschrauben 1 stark variierte und keine definierten Eigenschaften garantiert werden konnten. Für den Chirurgen bestand somit stets die Gefahr, eine minderqualitative bzw. für den konkreten Einsatz ungeeignete Knochenschraube 1 zu verwenden.

Mithilfe der Erfindung gelingt es Knochenschrauben 1 aus allogenem, kortikalen Knochen so auszuführen, dass Mindestwerte für ihre Biege- und Scherfestigkeit, sowie ihr Bruchdrehmoment sichergestellt werden. Das erfindungsgemäße Kriterium basierend auf dem Flächenverhältnis der Havers-Kanäle 6 zur Gesamtquerschnittsfläche der Knochenschraube 1 stellt dabei nicht nur eine überraschend einfache, sondern auch effektive Maßnahme dar, um die Festigkeit der Knochenschraube 1 zu kontrollieren.

## Patentansprüche

1. Bolzen mit Außengewinde und einem zylindrischen Bolzenschaft aus allogenem, kortikalen Knochenmaterial für die chirurgisch operative Osteosynthese, wobei das Knochenmaterial mit Havers-Kanälen (6) durchzogen ist, **dadurch gekennzeichnet, dass** in einem Querschnitt normal zur Längsachse (L) des Bolzenschaftes die Gesamtfläche der Havers-Kanäle (6) höchstens 10% der Gesamtfläche des Querschnittes einnimmt.

2. Verfahren zur Herstellung eines Bolzens mit Außengewinde und einem zylindrischen Bolzenschaft aus allogenem, kortikalen und mit Havers-Kanälen (6) durchzogenen Knochenmaterial eines Spenderknochens (8) für die chirurgisch operative Osteosynthese, wobei in einem ersten Schritt der Spenderknochen (8) durch Schnitte quer zu seiner Längserstreckung in mehrere Teilstücke (T_{N}) unterteilt wird, aus denen in einem zweiten Verfahrensschritt der Bolzenschaft der Knochenschrauben (1) in einer, zu den Havers-Kanälen (6) im Wesentlichen parallelen Richtung geschnitten wird, und in einem dritten Schritt der Bolzenschaft mit einem Außengewinde versehen wird, **dadurch gekennzeichnet, dass** ausschließlich jene Teilstücke (T_{N}) dem zweiten Verfahrensschritt unterzogen werden, deren Grund- und Deckfläche eine Gesamtfläche der Havers-Kanäle (6) von höchstens 10% der Gesamtfläche der jeweiligen Grund- oder Deckfläche aufweist.

## Claims

1. A bolt with an external thread and a cylindrical bolt shank made of an allogenic, cortical bone material for surgical osteosynthesis, wherein Haversian canals (6) extend through the bone material, **characterized in that** the total area of the Haversian canals (6) takes up not more than 10% of the total cross-sectional area in a cross-section perpendicular to the longitudinal axis (L) of the bolt shank.

2. A method for producing a bolt having an external thread and a cylindrical bolt shank from an allogenic, cortical bone material of a donor bone (8) for surgical osteosynthesis, with Haversian canals (6) extending through said bone material, wherein in a first step the donor bone (8) is subdivided into several sections (T_{N}) by cuts transversely to its longitudinal extension, from which in a second method step the bolt shank of the bone screws (1) is cut in a direction substantially parallel to the Haversian canals (6), and in a third step the bolt shank is provided with an external thread, **characterized in that** only such sections (T_{N}) are subjected to the second method step whose base and cover area has a total area of the Haversian canals (6) of a maximum of 10% of the total area of the respective base or cover surface.

## Revendications

1. Boulon avec un filetage extérieur et un corps de boulon cylindrique en matériau osseux cortical allogène pour ostéosynthèse chirurgicale, dans lequel le matériau osseux est parcouru par des canaux de Havers (6), **caractérisé en ce que** dans une section perpendiculaire à l'axe longitudinal (L) du corps du boulon, l'aire totale des canaux de Havers (6) occupe au maximum 10 % de l'aire totale de la section.

2. Procédé pour fabriquer un boulon avec un filetage extérieur et un corps de boulon cylindrique en matériau osseux cortical allogène parcouru par des canaux de Havers (6), provenant d'un os donneur (8), pour ostéosynthèse chirurgicale, dans lequel, dans une première étape, l'os donneur (8) est découpé par des coupes perpendiculaires à son extension longitudinale en plusieurs morceaux (T_{N}) à partir desquels, dans une deuxième étape de procédé, le corps de boulon des vis à os (1) est coupé dans un sens sensiblement parallèle aux canaux de Havers (6) et, dans une troisième étape, le corps du boulon est muni d'un filetage extérieur, **caractérisé en ce que** seuls sont soumis à la deuxième étape de procédé les morceaux (T_{N}) dont la surface de base et de sommet présente une aire totale des canaux de Havers (6) représentant au maximum 10 % de l'aire de la surface de base et de sommet en question.
